# EUROPEAN PATENT SPECIFICATION

(11) **EP 1 512 424 B1**
(45) Date of publication and mention of the grant of the patent: **17.11.2010**
(21) Application number: 03775917.2
(22) Date of filing: 27.11.2003
(51) Int. Cl.: A61M 5/158, A61M 25/06

(54) **PROTECTOR FOR INDWELLING NEEDLE HAVING WINGS**
MITTEL ZUM SCHUTZ EINER VERWEILNADEL MIT FLÜGELN
ELEMENT PROTECTEUR POUR AIGUILLE A DEMEURE COMPRENANT DES AILETTES

(30) Priority: 03.12.2002 JP 2002351149
(43) Date of publication of application: 09.03.2005
(73) Proprietor: KABUSHIKI KAISHA TOP, Tokyo 120-0035 (JP)
(72) Inventor: SUZUKI, Hirohito, c/o KABUSHIKI KAISHA TOP, Tokyo 120-0035 (JP)
(74) Representative: Fenlon, Christine Lesley
(86) International application number: PCT/JP2003/015158
(87) International publication number: WO 2004/050149

(56) References cited:
- WO-A-99/12594
- WO-A1-90/03196
- WO-A2-03/045491
- JP-A- 4 180 772
- JP-A- 2000 342 687

## Description

### Technical field:

The present invention relates to a protector for a indwelling needle having wings used in a medical treatment, such as drip infusion.

### Background art:

Conventionally, a indwelling needle having wings comprising a hollow needle, a holding sleeve for holding the proximal end of the hollow needle, and a pair of wings formed integrally with the holding sleeve has been used in medical treatments, such as drip infusion. One who conducts drip infusion or the like folds one wing over the other and inserts the hollow needle into a blood vessel of a patient by holding the wings in the folded state. Then, he/she unfolds the wings and attaches them to the skin with an adhesive, thereby retaining the hollow needle. Then, drip infusion or the like for the patient can be conducted by means of the hollow needle. The used indwelling needle having wings is removed from the body of the patient and discarded. However, if the hollow needle is uncovered, one who discards the needle may touch the needle and become infected with a pathogenic fungi or the like.

Thus, conventionally, a protector for a indwelling needle having wings has been used which houses a used indwelling needle having wings to prevent the hollow needle thereof from being touched from the outside (see Japanese Patent Laid-Open No. 11-319086, for example). This type of protector for a indwelling needle having wings has a substantially cylindrical protector main body, one slit that is formed in the peripheral wall of the main body, extends in the longitudinal direction from the distal end of the main body and is longer than the hollow needle, and a wing housing slot that is formed by partially cutting the peripheral wall of the protector main body, extends in the peripheral direction thereof and is connected with the slit near the proximal end of the protector main body. The slit guides the folded wings sliding therethrough. Once the folded wings having been guided through the slit reach the wing housing slot, the wings are unfolded to move away from each other and fitted in the wing housing slot. In this way, the wing housing slot restricts the movement of the unfolded wings, keeping the hollow needle housed in the protector and open. Thus, the hollow needle can be prevented from being touched from the outside.

However, since the wings have to be folded when housing the indwelling needle having wings in the protector, and the handling of the indwelling needle having wings is disadvantageously bothersome when housing the needle in the protector.

To eliminate such a disadvantage, an object of the present invention is to provide a protector for a indwelling needle having wings which requires only a simple operation to house a indwelling needle having wings in a protector main body and can house a hollow needle thereof with reliability.

Another example of prior art needle protectors is known fromWO 99/12594 A1.

### Disclosure of the invention:

In order to attain the object described above, the present invention provides a protector for a indwelling needle having wings for housing a indwelling needle having wings having a hollow needle having a cutting edge at the distal end thereof, a holding sleeve for holding the proximal end of the hollow needle, and a pair of wings formed integrally with the holding sleeve, comprising: a cylindrical outer sleeve for housing the indwelling needle having wings with the wings of the indwelling needle having wings uncovered; further, as defined in the characterising part of claim 1, a pair of slits for guiding the wings in a housing direction and holding the wings at the proximal end thereof, the slits being formed in a peripheral wall of the outer sleeve, extending parallel to each other from the distal end of the outer sleeve to the proximal end thereof and being longer than the hollow needle; and at least a pair of projections that are provided across the slits and capable of resiliently pivoting toward the inside of the outer sleeve, and that one end of each projection which is to abut against the wing moving in the housing direction along the slit constitutes a slanted edge that obliquely traverses the slit, and the other end of each projection which is to abut against the wing located at the proximal end of the slit constitutes an engagement edge that locks the wing and restricts movement thereof in a removal direction.

According to the present invention, the wings can be grasped and pulled backward to be inserted into the slits without folding them. Furthermore, since one end of each projection constitutes the slanted edge, the projection is bent smoothly inward when the wing moving in the housing direction abuts against the inclined edge. Once the wings have moved to the proximal ends of the slits, the projections rise by a restoring force and get back to their original position. Since the other end of each projection constitutes the engagement edge for locking a wing, the wing is prevented by the projection frommoving in the longitudinal direction again. On the other hand, the hollow needle is moved backward together with the wings and housed in the protector. Thus, there is provided a protector for a indwelling needle having wings that allows the indwelling needle having wings to be housed in the protector main body in a simple operation.

According to the present invention, the projections are preferably provided inside the outer sleeve and have a size enough to traverse the slit. In this case, when a force that bends the projections outward is applied to the projections, the projections are engaged with the inner wall of the outer sleeve and block off the slits. Thus, the hollow needle can be prevented from protruding outward through the slits with reliability.

In addition, according to the present invention, a gutter-shaped member is provided in the outer sleeve, the gutter-shaped member being curved so as to conform to the inner wall surface of the outer sleeve and having side edges located near the slits, and the projections may be formed integrally with the side edges of the gutter-shaped member, thereby simplifying the structure of the protector.

In addition, according to the present invention, the one of the slits extends from the distal end of the outer sleeve to the proximal end thereof and is opened at both the ends, and a blocking piece is provided in the outer sleeve, the blocking piece blocking off the proximal end of the slit to prevent removal of the wing therethrough and being capable of resiliently pivoting toward the inside of the outer sleeve. In this case, even after a tube having the indwelling needle having wings at the distal end is equipped with an infusion fluid bag or the like at the proximal end, the tube can be fitted with the protector for a indwelling needle having wings according to the present invention through the slit.

In this case, a gutter-shaped member may be provided in the outer sleeve, the gutter-shaped member being curved so as to conform to the inner wall surface of the outer sleeve and having side edges located near the slits, the projections may be formed integrally with the side edges of the gutter-shaped member at positions close to the distal end of the gutter-shaped member, and the blocking piece may be formed integrally with one of the side edges of the gutter-shaped member at a position close to the proximal end of the gutter-shaped member, thereby simplifying the structure of the protector.

In addition, according to the present invention, a plurality of pairs of projections are preferably provided along the slits. In this case, each projection can have a reduced length along the slit. Thus, each projection can be quickly bent inward when it is pressed by the wing moving in the housing direction and can be quickly restored after the wing has passed it.

### Brief description of the drawings:

FIG. 1 is a general view of a protector for a indwelling needle having wings according to a first embodiment of the present invention;
FIG. 2 is an exploded view of the protector for a indwelling needle having wings according to the first embodiment;
FIG. 3 is a partially cut away view of an outer sleeve of the protector according to the first embodiment;
FIG. 4 shows a indwelling needle having wings housed in the protector for a indwelling needle having wings according to the first embodiment;
FIG. 5 illustrates a process in which projections according to the first embodiment are bent inward;
FIG. 6 is a general view of a protector for a indwelling needle having wings according to a second embodiment of the present invention; and
FIG. 7 is an exploded view of the protector for a indwelling needle having wings according to the second embodiment.

Best mode for carrying out the invention:

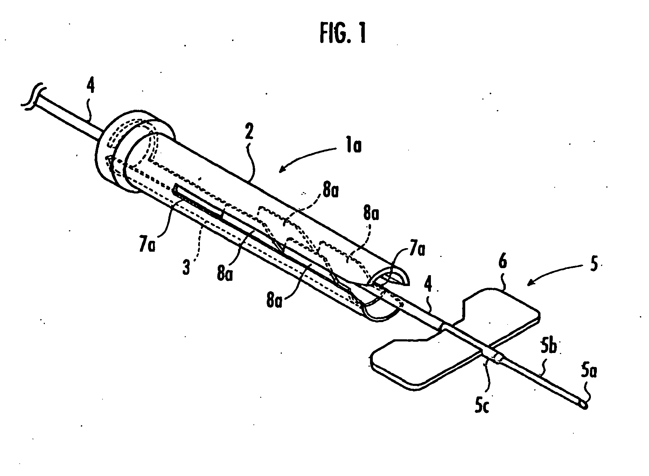

Embodiments of the present invention will be described with reference to FIGS. 1 to 7.

As shown in FIG. 1, a protector 1a for a indwelling needle having wings according to a first embodiment comprises an outer sleeve 2 and a gutter-shaped member 3. In the protector 1a for a indwelling needle having wings, a indwelling needle having wings 5 having a proximal end connected to a tube 4 is inserted in such a manner that it can be longitudinally displaced. The indwelling needle having wings 5 comprises a hollow needle 5b which is substantially cylindrical and has a cutting edge 5a at the tip, a substantially cylindrical holding sleeve 5c for holding the proximal end of the hollow needle 5b, and a pair of wings 6 which is made of a soft resin and is formed integrally with the holding sleeve 5c and diametrically opposed to each other with respect to the holding sleeve 5c. The hollow needle 5b is connected to medical equipment (not shown) via the tube 4.

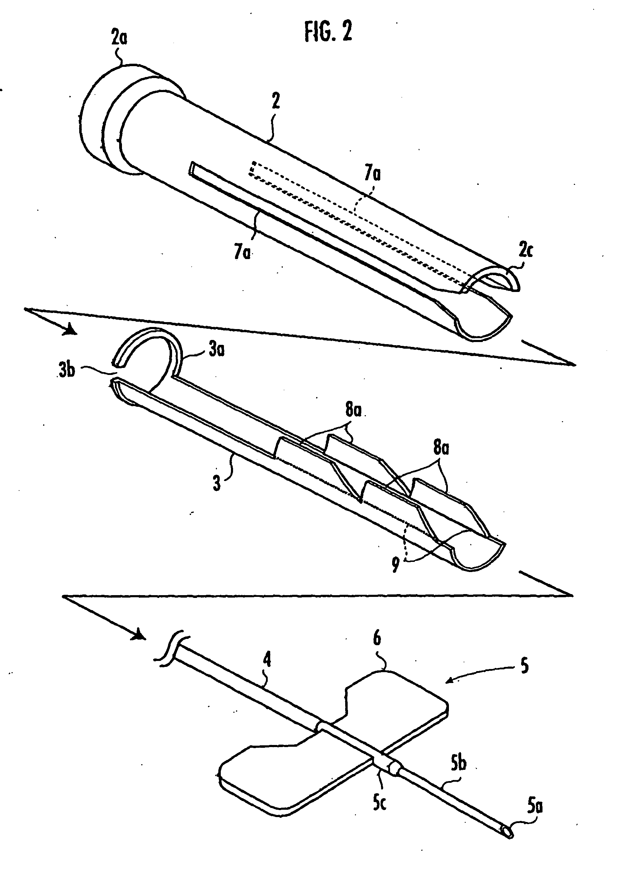

As shown in FIG. 2, the outer sleeve 2 has a pair of slits 7a, which are formed by cutting the peripheral wall of the sleeve in the longitudinal direction. The slits 7a extend from the distal end of the outer sleeve 2 to the middle thereof and is longer than the hollow needle. In addition, the wings 6 can be inserted to the slits 7a. The slits 7a have the distal ends tapered to facilitate guiding of the wings 6.

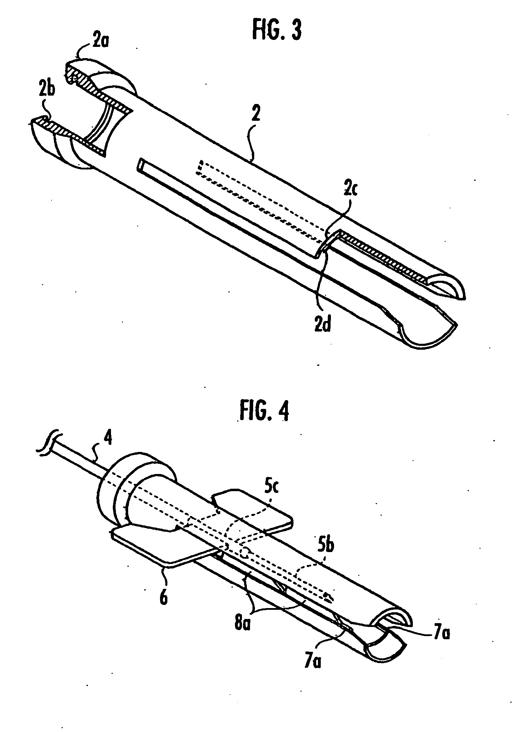

At the proximal end of the outer sleeve 2, a large-diameter part 2a having a larger outer diameter than the outer sleeve is formed. As shown in the partially cut away view of FIG. 3, in the inner wall of the large-diameter part 2a, an annular groove 2b is formed. In addition, an upper peripheral wall section 2c of the outer sleeve 2 has a larger thickness. This allows the inner wall of the outer sleeve 2 to have a step 2d.

As shown in FIG. 2, at the proximal end of the gutter- shaped member 3, a radially protruding annular member 3a is formed integrally with the gutter-shaped member. The annular member 3a has a gap 3b for the diameter to be readily reduced. The annular member 3a is fitted into the annular groove 2b of the outer sleeve 2 shown in FIG. 3. Besides, as shown in FIG. 1, the side edges of the gutter-shaped member 3 are located near the slits 7a. As shown in FIG. 2, on distal sections of the side edges of the gutter-shaped member 3, two pairs of projections 8a are provided side by side in the longitudinal direction and integrally with the side edges in such a manner that they can be bent inward at a recess 9. The distal end of each projection 8a in the longitudinal direction has a slanted edge that is slanted toward the side edge of the gutter-shaped member 3. The proximal end of each projection 8a in the longitudinal direction has an edge that is substantially perpendicular to the longitudinal direction and serves as an engagement edge in the first embodiment. When the indwelling needle having wings 5 is housed in the protector 1a for a indwelling needle having wings, the wings 6 are held in spaces defined by the projections 8a and the proximal ends of the slits 7, as shown in FIG. 4.

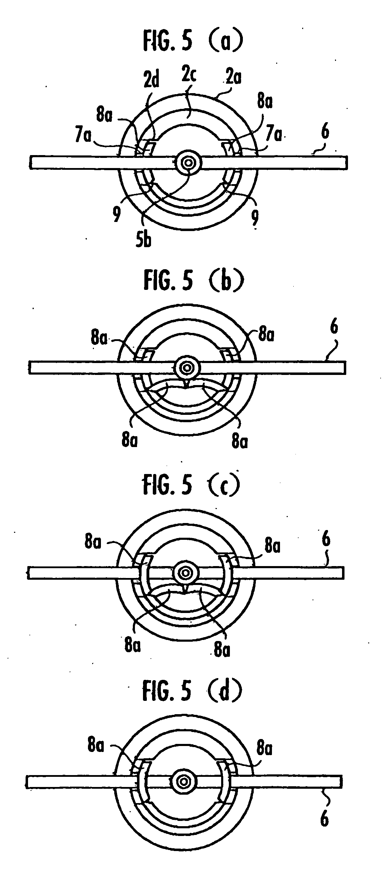

As shown in FIG. 5, the projections 8a are in contact with the inner wall of the outer sleeve 2 across the slits 7a. The upper edges of the projections 8a are engaged with the step 2d of the outer sleeve 2, thereby preventing the gutter-shaped member 3 from rotating about the axis. According to the first embodiment, since the upper edges of the projections 8a are engaged with the step 2d in this way, and the annular member 3a is fitted into the annular groove 2d, the gutter-shaped member 3 can be readily guided to and positioned at an installation position in the outer sleeve 2. The outer sleeve 2 and the gutter-shaped member 3 are bonded to each other with an adhesive, so that the gutter-shapedmember 3 is securely held in the outer sleeve 2.

Now, with reference to FIGS. 4 and 5, a process of housing the indwelling needle having wings 5 in the protector 1a for a indwelling needle having wings will be described. As shown in FIG. 5(a), one who conducts a medical treatment holds any of the wings 6 and pulls backward the same to insert the wings 6 into the slits 7a. Then, as shown in FIG. 5(b), the wings 6 are moved in the slits 7a while bending inward the distal pair of projections 8a. Then, as shown in FIG. 5(c), the wings 6 is further moved while bending inward the proximal pair of projections 8a, and the distal pairs of projections 8a rise by a restoring force. Then, as shown in FIGS. 4 and 5(d), the wings 6 are engaged with the proximal ends of the slits 7a, and the proximal pair of projections 8a also rise. Thus, the wings 6 are held in the spaces defined by the slits 7a and the projections 8a.

With the protector 1a for a indwelling needle having wings according to the first embodiment, the pair of wings 6 can be pulled backward to be inserted into the slits 7a without folding the wings. Furthermore, since the wings 6 are held in the spaces defined by the proximal ends of the slits 7a and the projections 8a, the wings 6 can be prevented from moving along the slits 7a toward the distal end of the protector under a certain level of power. That is, the operation of housing the wings indwelling needle in the protector main body is simplified, and the hollow needle can be housed in the protector with reliability.

Furthermore, the slits 7a are blocked off by the projections 8a, and the projections 8a are engaged with the inner wall of the outer sleeve 2 when a force that bends the projections 8a outward is applied thereto. Thus, the hollow needle 5b can be prevented from protruding from the slits 7a. In addition, since the protector 1a for a indwelling needle having wings according to the first embodiment comprises the outer sleeve 2 and the gutter-shaped member 3, the two components may be made of different materials. For example, the gutter-shaped member 3 may be made of a softer material than the outer sleeve 2. In addition, since separate two pairs of projections 8a, that is, the distal and proximal pairs of projections 8a, are formed, the projections 8a can be readily bent inward when housing the indwelling needle having wings 5 in the protector.

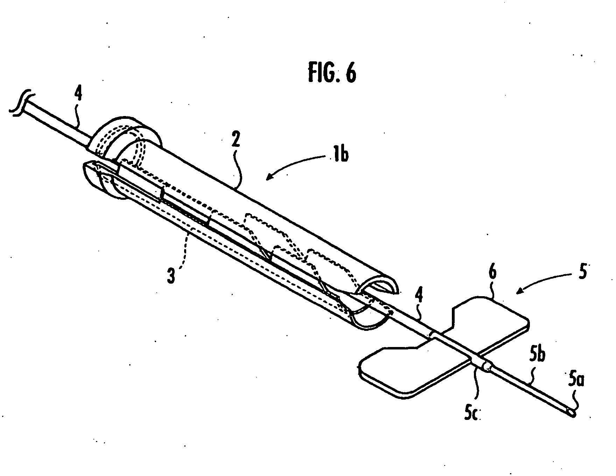

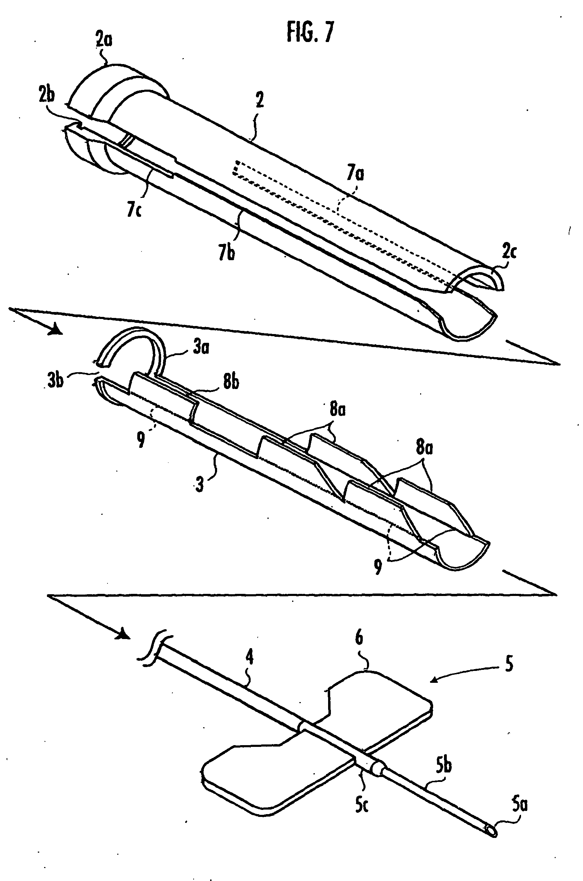

Now, with reference to FIGS. 6 and 7, a protector 1b for a indwelling needle having wings according to a second embodiment of the present invention will be described.

As shown in FIG. 6, the protector 1b for a indwelling needle having wings according to the second embodiment comprises an outer sleeve 2 and a gutter-shaped member 3, as with the protector 1a for a indwelling needle having wings according to the first embodiment. In addition, as shown in the exploded view of FIG. 7, one of slits 7b extends in the longitudinal direction from the distal end of the outer sleeve to the proximal end thereof. At a proximal section of the slit 7b, there is formed a wider section 7c for holding a tube 4 to fix the position of the protector 1b for a indwelling needle having wings.

An annular member 3a of the gutter-shaped member 3 has a gap 3b so as not to obstruct the wider section 7c. In addition, oneof the side edges of the gutter-shapedmember 3 has a blocking piece for blocking off a distal part of the wider section 7c via a recess 9. The blocking piece 8b can be bent inward owing to the recess 9. Furthermore, the upper edge of the blocking piece 8b is in contact with the inner wall of the outer sleeve 2, and thus, the blocking piece 8b is prevented from being bent outward with a higher reliability. Except for the points described above, the protector 1b for a indwelling needle having wings according to the second embodiment is the same as the protector 1a for a indwelling needle having wings according to the first embodiment.

With the protector 1b for a indwelling needle having wings according to the second embodiment, even after the tube 4 having the indwelling needle having wings 5 at the distal end is equipped with an infusion fluid bag or the like at the proximal end, the tube 4 can be fitted with the protector 1b for a indwelling needle having wings through the slit 7b.

### Industrial applicability:

According to the present invention, the indwelling needle having wings can be housed in the protector main body in a simple operation, and the hollow needle thereof can be housed in the protector with reliability. Therefore, using the protector in infusion or other medical treatments can allow the indwelling needle having wings to be readily removed from the body of a patient.

## Claims

1. A protector (1a) for an indwelling needle having wings for housing an indwelling needle having wings having a hollow needle having a cutting edge at the distal end thereof, a holding sleeve for holding the proximal end of the hollow needle, and a pair of wings formed integrally with the holding sleeve, comprising:
a cylindrical outer sleeve (2) for housing the indwelling needle having wings with the wings of the indwelling needle having wings uncovered;
the protector being **characterized by**:
a pair of slits (7a) for guiding the wings in a housing direction and holding the wings at the proximal end thereof, the slits (7a) being formed in a peripheral wall of the outer sleeve (2), extending parallel to each other from the distal end of the outer sleeve (2) to the proximal end thereof and being longer than the hollow needle; and
at least a pair of projections (8a) that are provided across the slits (7a) and capable of resiliently pivoting toward the inside of the outer sleeve (2).
wherein one end of each projection (8a) which is to abut against the wing moving in the housing direction along the slit (7a) constitutes a slanted edge that obliquely traverses the slit (7a), and the other end of each projection (8a) which is to abut against the wing located at the proximal end of the slit (7a) constitutes an engagement edge that locks the wing and restricts movement thereof in a removal direction.

2. The protector for an indwelling needle having wings according to claim 1, wherein said projections (8a) are provided inside the outer sleeve (2) and have a size enough to traverse the slit (7a).

3. The protector for an indwelling needle having wings according to claim 1, further comprising a gutter-shaped member (3) in said outer sleeve (2), the gutter-shaped member (3) being curved so as to conform to the inner wall surface of the outer sleeve (2) and having side edges located near the slits (7a),
wherein said projections (8a) are formed integrally with the side edges of the gutter-shaped member (3).

4. The protector for an indwelling needle having wings according to claim 1, wherein one of the slits (7a) extends from the distal end of said outer sleeve (2) to the proximal end thereof and is opened at both the ends, and
a blocking piece (8b) is provided in said outer sleeve, the blocking piece blocking off the proximal end of the slit (7a) to prevent removal of the wing therethrough and being capable of resiliently pivoting toward the inside of the outer sleeve (2).

5. The protector for an indwelling needle having wings accordingtoclaim4, further comprising a gutter-shaped member (3) in said outer sleeve, the gutter-shaped member (3) being curved so as to conform to the inner wall surface of the outer sleeve (2) and having side edges located near the slits (7a),
wherein said projections (8a) are formed integrally with the side edges of the gutter-shaped member (3) at the distal portion of the gutter-shaped member (3), and said blocking piece (8b) is formed integrally with one of the side edges of the gutter-shaped member (3) at the proximal portion of the gutter-shaped member.

6. The protector for an indwelling needle having wings according to claim 1, wherein a plurality of the projections (8a) are provided along the slit (7a).

## Patentansprüche

1. Schutz (1a) für einen Verweil-Flügelkatheter zum Aufnehmen eines Verweil-Flügelkatheters, der eine Kanüle mit einer Stichkante an ihrem distalen Ende, eine Haltemanschette zum Halten des proximalen Endes der Kanüle und ein Paar Flügel aufweist, die einstückig mit der Haltemanschette gebildet sind, umfassend:
eine zylindrische äußere Manschette (2) zum Aufnehmen des Verweil-Flügelkatheters, wobei die Flügel des Verweil-Flügelkatheters unbedeckt sind;
wobei der Schutz **gekennzeichnet ist durch**:
ein Paar Schlitze (7a), mit dem die Flügel in Richtung des Gehäuses geführt und an dessen proximalem Ende gehalten werden, wobei die Schlitze (7a) in einer Außenwand der äußeren Manschette (2) gebildet sind, sich parallel zueinander vom distalen Ende der äußeren Manschette (2) zu deren proximalem Ende erstrecken und länger als die Kanüle sind; und
mindestens ein Paar Fortsätze (8a) über den Schlitzen (7a), die elastisch zur Innenseite der äußeren Manschette (2) schwenken können,
wobei ein Ende von jedem Fortsatz (8a), das an den Flügel anstoßen soll, der in Richtung des Gehäuses den Schlitz (7a) entlang bewegt wird, eine abgeschrägte Seite bildet, die schräg über den Schlitz (7a) verläuft, und das andere Ende von jedem Fortsatz (8a), das an den Flügel anstoßen soll, der sich am proximalen Ende des Schlitzes (7a) befindet, eine Einrastseite bildet, die den Flügel arretiert und seine Bewegung in Entnahmerichtung behindert.

2. Schutz für einen Verweil-Flügelkatheter nach Anspruch 1, wobei die Fortsätze (8a) im Inneren der äußeren Manschette (2) bereitgestellt werden und genügend groß sind, dass sie über den Schlitz verlaufen.

3. Schutz für einen Verweil-Fiügelkatheter nach Anspruch 1, der zudem ein rinnenförmiges Element (3) in der äußeren Manschette umfasst, wobei das rinnenförmige Element (3) so gebogen ist, dass es an die Oberfläche der Innenwand der äußeren Manschette (2) angepasst ist, und Seitenränder hat, die sich in der Nähe der Schlitze (7a) befinden;
wobei die Fortsätze (8a) einstückig mit den Seitenrändern des rinnenförmigen Elements (3) gebildet sind.

4. Schutz für einen Verweil-Flügelkatheter nach Anspruch 1, wobei einer der Schlitze (7a) vom distalen Ende der äußeren Manschette (2) zu deren proximalem Ende verläuft und an beiden Enden offen ist und
ein Blockierstück (8b) in der äußeren Manschette bereitgestellt wird, wobei das Blockierstück das proximale Ende des Schlitzes (7a) blockiert, so dass der Flügel **dadurch** nicht entnommen werden kann, und elastisch zu Innenseite der äußeren Manschette (2) schwenken kann.

5. Schutz für einen Verweil-Flügelkatheter nach Anspruch 4, der zudem ein rinnenförmiges Element (3) in der äußeren Manschette umfasst, wobei das rinnenförmige Element (3) so gebogen ist, dass es an die Oberfläche der Innenwand der äußeren Manschette (2) angepasst ist, und Seitenränder hat, die sich in der Nähe der Schlitze (7a) befinden;
wobei die Fortsätze (8a) einstückig mit den Seitenrändern des rinnenförmigen Elements (3) an dem distalen Abschnitt des rinnenförmigen Elements (3) gebildet sind und das Blockierstück (8b) einstückig mit einem der Seitenränder des rinnenförmigen Elements (3) an dessen proximalem Abschnitt gebildet ist.

6. Schutz für einen Verweil-Flügelkatheter nach Anspruch 1, wobei eine Anzahl von Fortsätzen (8a) entlang der Schlitze (7a) bereitgestellt wird.

## Revendications

1. Élément protecteur (1a) pour aiguille à demeure comportant des ailettes pour loger une aiguille à demeure, comportant des ailettes comportant une aiguille creuse, comportant un bord de coupe à son extrémité distale, un manchon de maintien pour maintenir l'extrémité proximale de l'aiguille creuse, et une paire d'ailettes formées d'un seul tenant avec le manchon de maintien, comprenant :
un manchon extérieur cylindrique (2) pour loger l'aiguille à demeure comportant des ailettes, les ailettes de l'aiguille à demeure comportant des ailettes découvertes :
l'élément protecteur étant **caractérisé par** :
une paire de fentes (7a) pour guider les ailettes dans la direction du logement et maintenir les ailettes à son extrémité proximale, les fentes (7a) étant formées dans une paroi périphérique du manchon extérieur (2), s'étendant parallèlement entre elles depuis l'extrémité distale du manchon extérieur (2) jusqu'à son extrémité proximale et étant plus longues que l'aiguille creuse ; et
au moins une paire de projections (8a) prévues d'un bout à l'autre des fentes (7a) et capables de pivoter de manière élastique vers l'intérieur du manchon extérieur (2),
dans lequel une extrémité de chaque projection (8a), destinée à venir en butée contre l'ailette se déplaçant dans la direction du logement le long de la fente (7a), constitue un bord incliné qui traverse obliquement la fente (7a) et l'autre extrémité de chaque projection (8a), destinée à venir en butée contre l'ailette située à l'extrémité proximale de la fente (7a), constitue un bord d'engagement qui bloque l'ailette et limite son mouvement dans la direction du retrait.

2. Élément protecteur pour aiguille à demeure comportant des ailettes selon la revendication 1, dans lequel lesdites projections (8a) sont disposées à l'intérieur du manchon extérieur (2) et ont une taille suffisante pour traverser la fente (7a).

3. Élément protecteur pour aiguille à demeure comportant des ailettes selon la revendication 1, comprenant en outre un élément en forme de gouttière (3) dans ledit manchon extérieur (2), l'élément en forme de gouttière (3) étant incurvé de façon à se conformer la surface de la paroi intérieure du manchon extérieur (2) et ayant des bords latéraux situés près des fentes (7a),
dans lequel lesdites projections (8a) sont formées d'un seul tenant avec les bords latéraux de l'élément en forme de gouttière (3).

4. Élément protecteur pour aiguille à demeure comportant des ailettes selon la revendication 1, dans lequel l'une des fentes (7a) s'étend depuis l'extrémité distale dudit manchon extérieur (2) jusqu'à son extrémité proximale et est ouverte aux deux extrémités, et
une pièce de blocage (8b) est prévues dans ledit manchon extérieur, la pièce de blocage bloquant l'extrémité proximale de la fente (7a) pour empêcher le retrait de l'ailette à travers celle-ci et étant capable de pivoter de manière élastique vers l'intérieur du manchon extérieur (2).

5. Élément protecteur pour aiguille à demeure comportant des ailettes selon la revendication 4, comprenant en outre un élément en forme de gouttière (3) dans ledit manchon extérieur, l'élément en forme de gouttière (3) étant incurvé pour se conformer à la surface de la paroi intérieure du manchon extérieur (2) et ayant des bords latéraux situés près des fentes (7a),
dans lequel lesdites projections (8a) sont formées d'un seul tenant avec les bords latéraux de l'élément en forme de gouttière (3) au niveau de la partie distale de l'élément en forme de gouttière (3), et ladite pièce de blocage (8b) est formée d'un seul tenant avec l'un des bords latéraux de l'élément en forme de gouttière (3) au niveau de la partie proximale de l'élément en forme de gouttière.

6. Élément protecteur pour aiguille à demeure comportant des ailettes selon la revendication 1, dans lequel une pluralité de projections (8a) sont prévues le long de la fente (7a).
